# EUROPÄISCHE PATENTSCHRIFT

(11) **EP 1 616 890 B1**
(45) Veröffentlichungstag und Bekanntmachung des Hinweises auf die Patenterteilung: **30.11.2016**
(21) Anmeldenummer: 05013448.5
(22) Anmeldetag: 22.06.2005
(51) Int. Cl.: C07C 209/78, C07C 211/50, C07C 263/10, C07C 265/14, C08G 18/76

(54) **Verfahren zur Herstellung von Polyaminen der Diphenylmethanreihe bei niedriger Protonierung**
Method for producing of polyamines of the diphenylmethane series with low degree of protonation
Procédé de préparation de polyamines de la série du diphénylméthane par faible protonation

(30) Priorität: 05.07.2004 DE 102004032416
(43) Veröffentlichungstag der Anmeldung: 18.01.2006
(73) Patentinhaber: Covestro Deutschland AG, 51373 Leverkusen (DE)
(72) Erfinder: Hagen, Torsten, Dr., 45257 Essen (DE); Koch, Daniel, Dr., 47137 Duisburg (DE); Pirkl, Hans-Georg, Dr., 51377 Leverkusen (DE); Pohl, Fritz, Dr., 25541 Brunsbüttel (DE); Wershofen, Stefan, Dr., 41065 Mönchengladbach (DE); Uchdorf, Rudolf, 47829 Krefeld (DE); Adamson, Richard, 42799 Leichlingen (DE)
(74) Vertreter: Levpat

(56) Entgegenhaltungen:
- EP-A- 1 344 766
- EP-A- 1 375 561
- EP-A1- 0 158 059

## Beschreibung

Die Erfindung betrifft ein Verfahren zur Herstellung von Polyaminen der Diphenylmethanreihe.

Unter Polyaminen der Diphenylmethanreihe werden Verbindungen und Verbindungsgemische folgenden Typs verstanden: 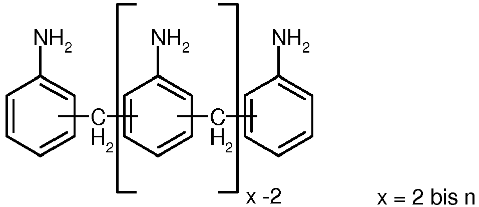 wobei n für eine natürliche Zahl > 2 steht.

Die kontinuierliche, diskontinuierliche oder halbkontinuierliche Herstellung von Polyaminen der Diphenylmethanreihe, nachfolgend auch als MDA bezeichnet, ist in zahlreichen Patenten und Publikationen beschrieben (siehe z.B. H.J. Twitchett, Chem. Soc. Rev. 3(2), 209 (1974), M.V. Moore in: Kirk-Othmer Encycl. Chem. Technol., 3rd. Ed., New York, 2, 338-348 (1978)). Üblicherweise erfolgt die Herstellung durch Umsetzung von Anilin und Formaldehyd in Anwesenheit saurer Katalysatoren. Üblicherweise wird HCl als saurer Katalysator eingesetzt. Der saure Katalysator wird gemäß dem Stand der Technik zum Ende des Prozesses und vor den abschließenden Aufarbeitungsschritten (wie beispielsweise der destillativen Entfernung von überschüssigem Anilin) durch Zusatz einer Base neutralisiert und somit verbraucht.

EP 1 375 561 A1 beschreibt ein Verfahren zur Herstellung von Polyaminen der Diphenylmethanreihe, bei dem man zunächst Anilin und Formaldehyd in Gegenwart eines sauren Katalysators zu Polyaminen umsetzt und danach das Reaktionsgemisch in Gegenwart eines Alkohols neutralisiert und/oder das Reaktionsgemisch nach der Neutralisation mit einem Alkohol versetzt und/oder nach der Neutralisation die Phasen trennt und die organische Phase mit einer Base und einem Alkohol versetzt, wobei das molare Verhältnis des Alkohols zum eingesetzten Formaldehyd mindestens 0,02 beträgt.

EP 1 344 766 A2 beschreibt ein Verfahren zur Herstellung von Polyaminen der Diphenylmethanreihe, bei dem man zunächst Anilin und Formaldehyd in Gegenwart eines sauren Katalysators zu Polyaminen umsetzt und danach das Reaktionsgemisch mit einer Base neutralisiert, nach der Neutralisation die wässrige und die organische Phase trennt und die organische Phase mit einer Base versetzt.

EP 0 158 059 A1 beschreibt ein Verfahren zur Herstellung von Polyaminen der Diphenylmethanreihe mit einem hohen Diamingehalt und niedrigem Gehalt an 2,2'-Diaminodiphenylmethan durch zweistufige Kondensation von Anilin mit Formaldehyd in Gegenwart von Salzsäure als Katalysator, wobei in der ersten Reaktionsstufe 2,0 bis 3,5 Mol Anilin pro Mol Formaldehyd unter Einhaltung eines Protonierungsgrades von 40 bis 60 % und einer Temperatur von unter 50°C zum Einsatz gelangen, und wobei in der zweiten Reaktionsstufe dem Reaktionsgemisch so viel weiteres Anilin hinzugefügt wird, dass das molare Verhältnis von Aminstickstoffatomen zu in Form von Methylenbrücken chemisch gebundenem Formaldehyd auf über 10:1 und bis zu 20:1 ansteigt und der Protonierungsgrad entsprechend abfällt.

Es ist allgemein bekannt, dass die saure Kondensation von aromatischen Aminen und Formaldehyd zu Polyaminen der Diphenylmethanreihe in mehreren Reaktionsschritten abläuft, wie exemplarisch im nachstehenden Schema erläutert:

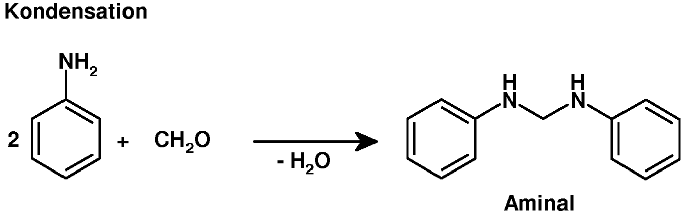

### 1. Umlagerung:

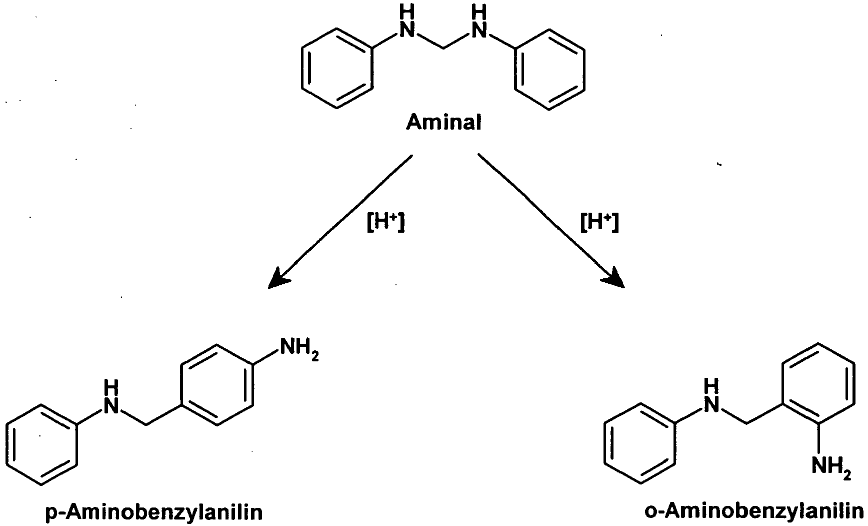

### 2. Umlagerung:

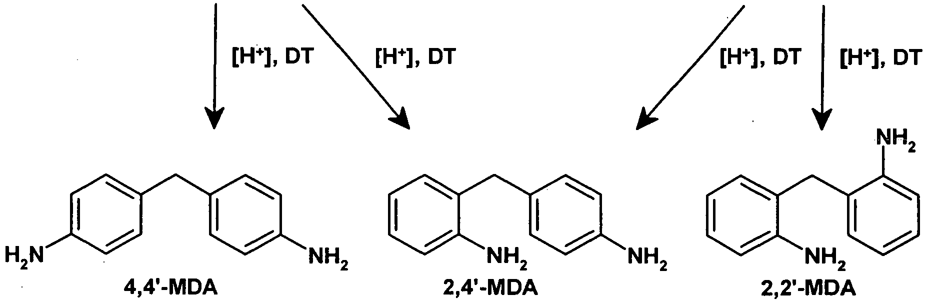

In Abwesenheit eines sauren Katalysators kondensiert zunächst Formaldehyd mit Anilin zu sogenanntem Aminal und Wasser. Die Umlagerung zum MDA erfolgt säurekatalysiert in einem ersten Schritt zu para- bzw. ortho-Aminobenzylanilin. Die Aminobenzylaniline lagern in einem zweiten Schritt zum MDA um. Hauptprodukte der säurekatalysierten Reaktion von Anilin und Formaldehyd sind das Diamin 4,4'-MDA, seine Stellungsisomere 2,4'-MDA und 2,2'-MDA sowie höhere homologe Polyamine der Diphenylmethanreihe.

In Anwesenheit eines sauren Katalysators entstehen aus Anilin und Formaldehyd direkt die Aminobenzylaniline, die anschließend weiter zu den zweikernigen MDA-Isomeren und höherkernigen MDA-Homologen reagieren.

Die Polyisocyanate der Diphenylmethanreihe, nachfolgend als MDI bezeichnet, werden durch Phosgenierung der entsprechenden Polyamine hergestellt. Die so hergestellten Polyisocyanate der Diphenylmethanreihe enthalten dabei die verschiedenen Isocyanat-Isomeren und deren höhere Homologe in der gleichen Zusammensetzung wie die Polyamine, aus denen sie hergestellt wurden.

Steuergröße zur Beeinflussung der Isomerenverteilung bei der säurekatalysierten. Reaktion von Anilin und Formaldehyd ist neben dem molaren Verhältnis von Anilin und Formaldehyd die in den Prozess eingesetzte Menge an saurem Katalysator. In diesem Zusammenhang wird als Protonierungsgrad bei einprotonigen Säuren wie HCl das molare Verhältnis aus eingesetztem sauren Katalysator und der im Reaktionsgemisch vorhandenen molaren Menge an Aminfunktionen bezeichnet. Um MDA mit der gewünschten Isomerenverteilung herstellen zu können, müssen daher zum Teil erhebliche Mengen an saurem Katalysator (entspricht einem hohen Protonierungsgrad) und entsprechend erhebliche Mengen an Base für die Neutralisation des sauren Katalysators eingesetzt werden.

So beschreibt z.B. DE-A-1 643 449 die Herstellung von MDA mit einem hohen Anteil an 4,4'-MDA durch Umsetzung von zuvor mit Säure umgesetztem Anilin mit Formaldehyd, wobei der Protorierungsgrad mindestens 25 %, vorzugsweise mindestens 50 %, und noch besser 75 bis 100 % beträgt. Dabei ist es erforderlich, den sauren Katalysator in einer solchen Menge einzusetzen, dass die Durchführung der Reaktion in einer einheitlichen Phase erfolgt. Sowohl Reaktionswasser als auch das den Einsatzstoffen entstammende Wasser verbleiben im Reaktionsgemisch.

DE-A-10 111 337 beschreibt die Herstellung von MDA bei einem Protonierungsgrad von < 20 %, wobei allerdings erfindungsgemäß MDA mit einem erhöhten Anteil an 2,4'-Isomerem erhalten wird. Auch hier verbleiben Reaktionswasser und das den Einsatzstoffen entstammende Wasser im Reaktionsgemisch.

Weiterhin ist bekannt, dass sich die Isomeren- und Homologenverteilung des MDA durch Modifizierung des Verweilzeit- und Temperaturprofils in Grenzen steuern lassen. Wie in EP-A-10 53 222 beschrieben, ist eine hohe Temperatur dann vorteilhaft, wenn bevorzugt 2,4'-MDA und 2,2'-MDA-reiche Typen hergestellt werden sollen.

Aus dem Stand der Technik wird gefolgert, dass der steigende Anfall an 2,4'-MDA bei niedrigem Protonierungsgrad durch niedrige Temperaturen in der Umlagerung zumindest teilweise vermieden werden kann. Ein niedriger Protonierungsgrad bedeutet aber einen geringeren Verbrauch an saurem Katalysator und auch einen geringeren Verbrauch an Base, um den sauren Katalysator im Anschluss an die Kondensationsreaktion zu neutralisieren. Eine Fahrweise mit niedrigem Protonierungsgrad ist daher besonders wirtschaftlich.

Die untere Grenze der technisch realisierbaren Protonierung wird jedoch überraschenderweise nicht durch Grenzen in der Einstellung von Temperatur- und/oder Verweilzeit markiert. Vielmehr wird bei den nach dem Stand der Technik beschriebenen Verfahren beobachtet, dass die sauren Reaktionsgemische bei der Umsetzung unterhalb eines Protonierungsgrades von 25 % heterogen bzw. zweiphasig werden (DE-A-198 04 918). Diese Zweiphasigkeit führt zu Problemen bei der Reaktionsführung.

Aufgabe der vorliegenden Erfindung war es daher, ein einfaches und wirtschaftliches Verfahren zur Herstellung von Polyaminen der Diphenylmethanreihe bereit zu stellen, mit dem der saure Katalysator mit Protonierungsgraden <15% eingesetzt werden kann, ohne dass sich das Reaktionsgemisch in ein zweiphasiges System entmischt und mit dem gleichzeitig Polyamine mit niedrigen Isomeren-Verhältnissen an 2,4'-MDA zu 4,4'-MDA hergestellt werden können.

Die Aufgabe wird erfindungsgemäß gelöst durch ein Verfahren zur sauer katalysierten Herstellung von Polyaminen der Diphenylmethanreihe gemäß Anspruch 1.

Die Erfindung betrifft ein Verfahren, bei dem man in Schritt a) Anilin und Formaldehyd zunächst in Abwesenheit des sauren Katalysators zu dem Aminal umsetzt und das Aminal anschließend mit saurem Katalysator versetzt und es bei Temperaturen von 30°C bis 95°C und bei Wassergehalten des so erhaltenen sauren Reaktionsgemisches von 0 bis 20 Gew.-% weiter umsetzt.

Erfindungsgemäß wird in Schritt a) nach der Umsetzung zum Aminal zunächst das Wasser zumindest teilweise aus dem Aminal entfernt, wobei man einen Wassergehalt von 0 bis 5 Gew.-% im Aminal einstellt, und anschließend das Aminal mit saurem Katalysator versetzt und bei Temperaturen von 30°C bis 95°C und bei Wassergehalten des so erhaltenen sauren Reaktionsgemisches von 0 bis 20 Gew.-% weiter umgesetzt.

Das erfindungsgemäße Verfahren kann sowohl kontinuierlich, halbkontinuierlich als auch diskontinuierlich durchgeführt werden. Verwendung finden dabei die im Stand der Technik üblichen Reaktionsapparate. Geeignet sind zum Beispiel gerührte Reaktoren, Rohrreaktoren oder auch Rohrreaktoren mit Einbauten wie Lochböden, welche die Verweilzeitcharakteristik im Reaktor beeinflussen. Geeignet ist auch eine Kombination aus mehreren Reaktortypen.

Mit dem erfindungsgemäßen Verfahren können Polyamine der Diphenylmethanreihe bei Protonierungsgraden von < 15%, bevorzugt 4 bis 14%, besonders bevorzugt 5 bis 13% hergestellt werden. Als Protonierungsgrad wird dabei bei einprotonigen sauren Katalysatoren (wie Salzsäure) das molare Verhältnis aus der Menge an eingesetztem sauren Katalysator und der im Reaktionsgemisch vorhandenen molaren Menge an Aminfunktionen bezeichnet. Bei zwei- und mehrprotonigen Säuren (n-protonig) wird entsprechend unter dem Protonierungsgrad das molare Verhältnis aus der doppelten bzw. n-fachen Menge an eingesetztem sauren Katalysator und der im Reaktionsgemisch vorhandenen molaren Menge an Aminfunktionen bezeichnet.

Geeignete Polyamingemische der Diphenylmethanreihe werden erhalten durch Kondensation von Anilin und Formaldehyd im molaren Verhältnis 1,5:1 bis 6:1, bevorzugt 1,8:1 bis 5:1.

Formaldehyd wird technisch üblicherweise als wässrige Lösung eingesetzt, die in Konzentrationen von 30-50 Gew.-% vorliegt. Es ist aber ebenfalls möglich, wässrige Formaldehydlösungen anderer Konzentration oder andere Methylengruppen liefernde Verbindungen, wie z.B. Polyoxymethylenglykol, para-Formaldehyd oder Trioxan einzusetzen.

Als saure Katalysatoren haben sich starke organische und vorzugsweise anorganische Säuren bewährt. Geeignete Säuren sind Salzsäure, Schwefelsäure, Phosphorsäure, und Methansulfonsäure. Bevorzugt wird im erfindungsgemäßen Verfahren wässrige Salzsäure eingesetzt. Die wässrige Salzsäure liegt dabei üblicherweise in Konzentrationen von 25 bis 36 Gew.-% vor.

Die Erfindung betrifft ein Verfahren, bei dem man Anilin und Formaldehyd zunächst in Abwesenheit des sauren Katalysators zum Aminal umsetzt und anschließend den sauren Katalysator zu dem Reaktionsgemisch gibt. Die Vermischung von Aminal mit saurem Katalysator erfolgt dabei bevorzugt mit einem spezifischen Leistungseintrag von größer 10 kW/m³ Mischraum, besonders bevorzugt von größer 20 kW/m³ Mischraum und bevorzugt bei Temperaturen von 20 bis 60°C. Die Zugabe des sauren Katalysators und die Entfernung des Wassers können beispielsweise so erfolgen, dass in einen das erzeugte Aminal enthaltenden Rührkessel wässrige HCl zugegeben wird und der größte Teil des Wassers während der Umsetzung zum Kondensationsprodukt durch destillative Abtrennung entfernt wird und ein Wassergehalt von 0 bis 20 Gew.-%, bezogen auf das saure Reaktionsgemisch, eingestellt wird.

Erfindungsgemäß entfernt man nach der Umsetzung zum Aminal hieraus zunächst das Wasser zumindest teilweise, wobei man einen Wassergehalt von 0 bis 5 Gew.-%, bezogen auf das Aminal, erhält. Anschließend vermischt man das Aminal mit saurem Katalysator, wobei die Vermischung bevorzugt bei Temperaturen von 20 bis 60°C erfolgt, und setzt das so erhaltene saure Reaktionsgemisch bei Temperaturen von 30°C bis 95°C und bei Wassergehalten von 0 bis 20 Gew.-%, bezogen auf das erhaltene saure Reaktionsgemisch, zum Kondensationsprodukt um. Die Vermischung von Aminal mit saurem Katalysator erfolgt dabei bevorzugt mit einem spezifischen Leistungseintrag von größer 10 kW/m³ Mischraum, besonders bevorzugt von größer 20 kW/m³ Mischraum. Dabei ergibt sich der Leistungseintrag z.B. bei einer Mischdüse aus dem Druckverlust der vorgeschalteten Druckpumpe und dem Volumen der Mischdüse.

Erfindungsgemäß werden zunächst Anilin und Formaldehyd in Abwesenheit des sauren Katalysators vermischt und bei Temperaturen von 20°C bis 100°C, bevorzugt von 40°C bis 100°C, besonders bevorzugt von 60°C bis 95°C vermischt und zur Reaktion gebracht. Hierbei bilden sich Kondensationsprodukte aus Anilin und Formaldehyd (sogenanntes Aminal). Im Anschluss an die Aminalbildung wird das in dem Aminal enthaltene Wasser beispielsweise durch Phasentrennung oder durch andere geeignete Verfahren, beispielsweise durch Destillation, zumindest teilweise entfernt. Dabei resultiert ein Reaktionsprodukt mit einem Wassergehalt von 0 bis 5 Gew.-%, bevorzugt 0,5 bis 5 Gew.-%, besonders bevorzugt 1 bis 4 Gew.-% bezogen auf das Aminal. Der Wassergehalt lässt sich beispielsweise mit der Methode nach Karl-Fischer ermitteln, die z.B. bei Jander, Jahr, Maßanalyse, 15. Aufl., de Gruyter, Berlin (1989), S. 289 bis S. 292 beschrieben ist. Das Aminal wird dann mit dem sauren Katalysator, bevorzugt bei Temperaturen von 20-60°C und mit spezifischen Leistungseinträgen von bevorzugt größer 10 kW/m³ Mischraum, besonders bevorzugt von größer 20 kW/m³ Mischraum, vermischt und in einem Verweilzeitapparat oder einer Abfolge von Verweilzeitapparaten bei 30°C bis 95°C einer Vorreaktion unterzogen. Der Wassergehalt des resultierenden sauren Reaktionsgemisches liegt dabei zwischen 0 und 20 Gew.-%, bezogen auf das saure Reaktionsgemisch. Dabei wird die Menge an saurem Katalysator so gewählt, dass ein Protonierungsgrad von < 15%, bevorzugt von 5 bis 14%, besonders bevorzugt von 6 bis 13% eingestellt wird.

In Schritt b) des erfindungsgemäßen Verfahrens wird die Temperatur des Reaktionsgemisches in Stufen oder kontinuierlich und gegebenenfalls unter Überdruck auf eine Temperatur von 110°C bis 250°C, bevorzugt auf 110°C bis 180°C, besonders bevorzugt auf eine Temperatur von 110°C bis 160°C gebracht. Die Temperaturerhöhung in Schritt b) erfolgt dabei erfindungsgemäß frühestens dann, wenn das Verhältnis aus den Gewichtsanteilen an p-Aminobenzylanilin und 4,4'-MDA im sauren Reaktionsgemisch einen Wert von 1,00, bevorzugt von 0,50, besonders bevorzugt 0,25, ganz besonders bevorzugt 0,20 unterschreitet.

Die Temperatur in Schritt b) wird in diesem Temperaturbereich bevorzugt für eine Verweilzeit von 20 bis 300 min, besonders bevorzugt 40 bis 200 min, ganz besonders bevorzugt 50 bis 180 min gehalten, um vollständigen Umsatz sicherzustellen. Die Einhaltung dieser Umsatz-/Temperaturgrenzen stellt sicher, dass das Gewichtsverhältnis der Isomeren 2,4'- und 4,4'-MDA im Produkt zwischen 0,05 und 0,15 beträgt.

Zur Ermittlung des Verhältnisses aus den Gewichtsanteilen an p-Aminobenzylanilin und 4,4'-MDA im sauren Reaktionsgemisch kann der Gewichtsanteil dieser Komponenten beispielsweise durch Analyse mittels HPLC an einer Umkehrphasensäule mittels Gradientenelution mit einem Lösungsmittelgemisch aus Methanol, Acetonitril und Wasser ermittelt werden. Die Detektion der Komponenten erfolgt dabei vorzugsweise durch einen UV-Detektor bei einer Wellenlänge von 254 nm. Dabei geht man beispielsweise so vor, dass man die Vorreaktion bei Temperaturen von 20 bis 100°C durchführt und dem sauren Reaktionsgemisch Proben entnimmt und diese auf das Gewichtsverhältnis von p-ABA zu 4,4'-MDA hin analysiert. Dabei ist dafür Sorge zu tragen, dass sich die Zusammensetzung des sauren Reaktionsgemisches zwischen der Probenentnahme und der Analyse nicht verändert. Dies lässt sich beispielsweise dadurch erreichen, dass das saure Reaktionsgemisch stark abgekühlt wird, beispielsweise auf Temperaturen unter 0°C, so dass die Reaktionsgeschwindigkeit so gering wird, dass sich die Zusammensetzung nicht mehr merklich ändert. Ebenfalls möglich ist es, das saure Reaktionsgemisch mit einer Base zu versetzen, so dass der Reaktion der saure Katalysator entzogen wird und diese dadurch zum Erliegen kommt. Bei diskontinuierlicher Fahrweise beispielsweise kann dem Reaktionsbehälter in beliebiger zeitlicher Abfolge oder auch in regelmäßigen Abständen von 60 min, bevorzugt von 30 min, eine Probe entnommen und analysiert werden. Unterschreitet das Verhältnis von p-ABA zu 4,4'-MDA einen Wert von 1,00, so kann auf Temperaturen von größer oder gleich 110°C aufgeheizt werden. Bei einer kontinuierlichen Fahrweise, in der beispielsweise die Vorreaktion bei 20 bis 100°C in mindestens einem Reaktionsapparat oder in einer Abfolge von Reaktionsapparaten stattfindet, können beispielsweise am Ausgang des letzten Reaktionsbehälters Proben entnommen und in oben genannter Weise analysiert werden.

Überschreitet das Verhältnis p-ABA zu 4,4'-MDA den Wert von 1,00, so ist durch geeignete Maßnahmen der Reaktionsverlauf so zu steuern, dass der Wert von 1,00 anschließend unterschritten wird. Beispielsweise kann der Durchsatz an saurer Reaktionslösung verringert werden, so dass durch die nun vermehrte Verweilzeit am Ausgang des letzten Reaktionsbehälters ein größerer Umsatz der Vorreaktion erzielt wird und das Verhältnis von p-ABA zu 4,4'-MDA nun kleiner als 1,00 ist. Es ist aber auch möglich, die Temperatur in dem zur Vorreaktion genutzten Reaktionsbehälter oder das Temperaturprofil in den zur Vorreaktion verwendeten Reaktionsbehältern derart anzupassen, dass am Ausgang des letzten Behälters das Verhältnis p-ABA zu 4,4'-MDA kleiner als 1,00 ist, sofern die Temperatur oder das Temperaturprofil innerhalb der Grenzen 20 bis 100°C gehalten wird.

Die Umsetzung von Anilin und Formaldehyd in Gegenwart eines sauren Katalysators zu Polyaminen der Diphenylmethanreihe kann in Anwesenheit weiterer Stoffe, z.B. von Salzen, organischen und anorganischen Säuren geschehen.

Durch das erfindungsgemäße Verfahren wird erreicht, dass trotz des niedrigen Protonierungsgrades von <15% keine Entmischung während der Umsetzung auftritt. Dies ist von Vorteil, da bei einem niedrigen Protonierungsgrad und gleichzeitig einem hohen Wassergehalt das saure Reaktionsgemisch in eine wässrige und eine organische Phase zerfällt, und der saure Katalysator dann zu großen Teilen in die wässrige Phase übergeht und der organischen Phase nicht mehr uneingeschränkt zur Verfügung steht und somit der Reaktion entzogen ist. Dies führt zum einen zu einer geringeren Reaktionsgeschwindigkeit, weshalb für die Reaktion mehr Verweilzeit benötigt wird. Dies beeinträchtigt aber die Wirtschaftlichkeit des Verfahrens. Zum anderen führt dies zu einer ungünstigen Produktzusammensetzung, denn dann wird beispielsweise mehr unerwünschtes 2,4'-MDA gebildet.

Gleichzeitig wird durch das erfindungsgemäße Verfahren erreicht, dass MDA im Gewichtsverhältnis der Isomeren 2,4'-MDA zu 4,4'-MDA von zwischen 0,05 und 0,15 erhalten werden kann. Das Verhältnis von p-Aminobenzylanilin zu 4,4'-MDA ist ein Maß für den erreichten Umsatz. Hohe Temperaturen während der Umsetzung des p-Aminobenzylanilins sowie anderer isomerer und höherkerniger Aminobenzylaniline begünstigen die Bildung der 2,2'- und 2,4'-MDA-Isomeren. Dies bedeutet, dass 2,4'-MDA in großen Mengen gebildet wird, wenn ein großer Teil des Umsatzes bei hoher Temperatur über 110°C erzielt wird. Umgekehrt gilt, dass weniger 2,2'- und 2,4'-MDA und dafür mehr 4,4'-MDA gebildet wird, wenn ein großer Teil des Umsatzes bei geringeren Temperaturen erreicht wird. Daher kann mit dem erfindungsgemäßen Verfahren die Bildung von 4,4'-MDA begünstigt und die Bildung von 2,2'- und 2,4'-MDA behindert werden, weil in dem erfindungsgemäßen Verfahren die Temperatur des Reaktionsgemisches erst dann auf 110 bis 250°C erhöht wird, wenn das Verhältnis aus den Gewichtsteilen von p-Aminobenzylanilin und 4,4'-MDA kleiner als 1,00 ist. So wird erreicht, dass das Gewichtsverhältnis der Isomeren 2,4'- und 4,4'-MDA im Produkt zwischen 0,05 und 0,15 beträgt.

Zur Aufarbeitung des sauren Reaktionsgemisches wird das Reaktionsgemisch mit einer Base gemäß dem Stand der Technik neutralisiert. Nach dem Stand der Technik erfolgt die Neutralisation üblicherweise bei Temperaturen von beispielsweise 90 bis 100°C ohne Zusatz weiterer Substanzen. (H.J. Twitchett, Chem. Soc. Rev. 3(2), 223 (1974)). Sie kann aber auch auf einem anderen Temperaturniveau erfolgen, um z.B. den Abbau von störenden Nebenprodukten zu beschleunigen. Als Basen sind beispielsweise geeignet die Hydroxide der Alkali- und Erdalkalielemente. Vorzugsweise kommt wässrige NaOH zur Anwendung.

Die zur Neutralisation eingesetzte Base wird in Mengen von größer 100%, vorzugsweise 105 bis 120% der für die Neutralisation des eingesetzten sauren Katalysators stöchiometrisch benötigten Menge eingesetzt.

Im Anschluss an die Neutralisation wird in einem Trennbehälter üblicherweise die organische von der wässrigen Phase getrennt. Die nach Abtrennung der wässrigen Phase verbleibende produktenthaltende organische Phase wird weiteren Aufarbeitungsschritten unterzogen (z.B. Wäsche) und anschließend von überschüssigem Anilin und anderen im Gemisch vorhandenen Stoffen (z.B. weiteren Lösungsmitteln) durch geeignete Verfahren wie z.B. Destillation, Extraktion oder Kristallisation befreit.

Das so erhaltene Polyamin oder Polyamingemisch der Diphenylmethanreihe kann nach den bekannten Methoden mit Phosgen in einem inerten organischen Lösungsmittel zu den entsprechenden Di- und Polyisocyanaten der Diphenylmethanreihe, dem MDI, umgesetzt werden. Das Molverhältnis von Roh-MDA zu Phosgen wird zweckmäßigerweise so bemessen, dass pro Mol NH₂-Gruppe 1 bis 10 Mol, vorzugsweise 1,3 bis 4 Mol Phosgen in der Reaktionsmischung vorliegen. Als inerte Lösungsmittel haben sich chlorierte, aromatische Kohlenwasserstoffe wie z.B. Monochlorbenzol, Dichlorbenzole, Trichlorbenzole, die entsprechenden Toluole und Xylole sowie Chlorethylbenzol bewährt. Insbesondere Anwendung finden als inerte organische Lösungsmittel Monochlorbenzol, Dichlorbenzol oder Mischungen dieser Chlorbenzole. Die Menge an Lösungsmittel wird zweckmäßigerweise so bemessen, dass die Reaktionsmischung einen Isocyanatgehalt von 2 bis 40 Gew.-%, vorzugsweise zwischen 5 uns 20 Gew.-%, bezogen auf das Gesamtgewicht der Reaktionsmischung, aufweist. Nach beendeter Phosgenierung wird aus der Reaktionsmischung das überschüssige Phosgen, das inerte organische Lösungsmittel oder Mischungen davon durch Destillation abgetrennt.

Aus dem gewonnenen Roh-MDI können die gemäß dem Stand der Technik bekannten Produkte der polymeren MDI-Reihe, enthaltend zwei- und höherkernige Di- und Polyisocyanate der Diphenylmethanreihe, sowie der monomeren MDI-Reihe, enthaltend zweikernige Diisocyanate der Diphenylmethanreihe, hergestellt werden, insbesondere hochviskose polymere MDI-Typen mit 80 bis 3.000 mPas bei 25°C, technisch reines 4,4'-MDI und/oder technisch reines 2,4'-MDI sowie deren Mischformen. Die Abtrennung dieser Produkte aus dem Roh-MDI kann durch Verfahren gemäß dem Stand der Technik, beispielsweise Destillation, erfolgen. Diese Produkte eignen sich für den Einsatz als Rohstoffe für die Polyurethan-Herstellung in Form von Polymeren und Prepolymeren durch Reaktion mit Polyolen.

Die Erfindung soll anhand der nachfolgenden Beispiele näher erläutert werden.

### Beispiele:

Zur Bestimmung der Zusammensetzung des Polyamingemisches wird dieses mittels eines HPLC 1050 der Firma Hewlett Packard durch Umkehrphasenchromatographie an einer C 18-Säule mittels Gradientenelution mit einem ternären Gemisch aus Methanol, Acetonitril und Wasser unter Verwendung eines UV-Detektors bei einer Wellenlänge von 254 nm getrennt. Die Komponenten p-ABA und 4,4'-MDA sind als deutlich separierte Einzelsignale zu erkennen. Proben aus dem sauren Reaktionsgemisch werden vor der Analyse neutralisiert.

### Beispiel 1 (nicht erfindungsgemäß, zweiphasiges Reaktionsgemisch):

Zu 670 g Anilin werden bei 80°C unter Rühren innerhalb von 20 min 310 g einer 31,7 Gew.-%igen wässrigen Formaldehydlösung getropft. Nach der Zugabe wird noch 5 min bei 80°C gerührt und anschließend auf 35°C abgekühlt. Bei dieser Temperatur wird das Reaktionsgemisch unter intensivem Rühren mit 83,0 g einer 31,9 Gew.-%igen wässrigen Salzsäure versetzt. Der Wassergehalt des sauren Reaktionsgemisches beträgt 30,7 Gew.-%. Das saure Reaktionsgemisch ist auch nach dem Aufheizen auf 110°C noch deutlich zweiphasig, d. h. ohne Rühreinwirkung tritt ein Zerfall der sauren Reaktionsmischung in eine wässrige und eine organische Phase ein.

### Beispiel 2 (nicht erfindungsgemäß, vollständiger Umsatz bei >110°C):

Zur Herstellung des Aminals werden zu 931 g Anilin bei 80°C unter Rühren innerhalb von 20 min 334 g einer 32,1 Gew.-%igen wässrigen Formaldehydlösung getropft. Nach der Zugabe wird noch 5 min bei 80°C gerührt und bei 70 bis 80°C eine Phasentrennung vorgenommen. 900 g der organischen Phase, dem Aminal, werden bei 115°C innerhalb von 10 min unter intensivem Rühren mit 137 g einer 32,0 Gew.-%igen wässrigen Salzsäure vermischt. Der Wassergehalt beträgt nach der Salzsäurezugabe 12,0 Gew.-%, das Verhältnis aus p-ABA und 4,4'-MDA 0,27. Nach weiteren 3 h Rühren bei 115°C sind sämtliche zweikernigen Aminobenzylaniline umgesetzt. Das Verhältnis aus 2,4'- und 4,4'-MDA beträgt 0,38. Dieses Beispiel demonstriert die Auswirkung auf die Produktzusammensetzung, wenn ein vollständiger Umsatz bei einer hohen Temperatur von >110°C erzielt wird.

### Beispiel 3 (erfindungsgemäß, größter Teil des Umsatzes bei 30 bis 95°C):

900 g Aminal, hergestellt gemäß Beispiel 2, wird bei 45°C innerhalb von 10 min unter intensivem Rühren mit 137 g einer 32,0 Gew.-%igen wässrigen Salzsäure vermischt. Der Wassergehalt beträgt nach der Salzsäurezugabe 12,0 Gew.-%, das entstehende Reaktionsgemisch zeigt ohne Rühreinwirkung keine Entmischung in eine wässrige und eine organische Phase. Nach 10 min Rühren bei 45°C wird auf 80°C aufgeheizt und weitere 60 min bei 80°C gerührt. Das Verhältnis p-ABA/4,4'-MDA beträgt 0,05. Anschließend wird auf 115°C aufgeheizt und weitere 3 h bei dieser Temperatur gerührt. Es sind keine zweikernigen Aminobenzylaniline mehr nachweisbar, das Verhältnis aus 2,4'- und 4,4'-MDA beträgt 0,14. Gegenüber Beispiel 2 ist eine deutliche Absenkung des Verhältnisses von 2,4'- zu 4,4'-MDA zu erkennen, da ein großer Teil des Umsatzes bei Temperaturen <110°C erzielt wurde.

## Patentansprüche

1. Verfahren zur Herstellung von Polyaminen der Diphenylmethanreihe, bei dem
a) zunächst Anilin und Formaldehyd im molaren Verhältnis von 1,5:1 bis 6:1 in Abwesenheit eines sauren Katalysators vermischt und bei Temperaturen von 20°C bis 100°C zur Reaktion gebracht werden, wobei sich Aminal bildet;
im Anschluss an die Aminalbildung das in dem Aminal enthaltene Wasser zumindest teilweise entfernt wird, wobei ein Reaktionsprodukt mit einem Wassergehalt von 0 bis 5 Gew.-%, bezogen auf das Aminal, resultiert;
das Aminal dann mit einem sauren Katalysator ausgewählt aus der Gruppe bestehend aus Salzsäure, Schwefelsäure, Phosphorsäure und Methansulfonsäure vermischt und bei 30°C bis 95°C einer Vorreaktion unterzogen wird, wobei der Wassergehalt des resultierenden sauren Reaktionsgemisches zwischen 0 und 20 Gew.-%, bezogen auf das saure Reaktionsgemisch, liegt, und wobei die Menge an saurem Katalysator so gewählt wird, dass ein Protonierungsgrad von < 15% eingestellt wird, wobei sich der Protonierungsgrad einer n-protonigen Säure aus dem molaren Verhältnis aus der n-fachen Menge an eingesetztem sauren Katalysator und der im Reaktionsgemisch vorhandenen molaren Menge an Aminfunktionen ergibt;
b) die Temperatur des Reaktionsgemisches auf eine Temperatur von 110°C bis 250°C gebracht wird, wenn das Verhältnis aus den Gewichtsanteilen an p-Aminobenzylanilin und 4,4'-MDA im sauren Reaktionsgemisch einen Wert von 1,00 unterschreitet.

2. Verfahren nach Anspruch 1, bei dem man in Schritt a) einen Protonierungsgrad von 5 bis 14% einstellt.

3. Verfahren nach einem der Ansprüche 1 oder 2, bei dem man in Schritt b) die Temperatur auf Werte von 110 °C bis 180 °C erhöht.

4. Verfahren zur Herstellung von Polyisocyanaten der Diphenylmethanreihe, bei dem man Polyamine der Diphenylmethanreihe nach dem Verfahren nach einem der Ansprüche 1 bis 3 herstellt und die Polyamine anschließend phosgeniert.

5. Verfahren zur Herstellung von Polyurethanen, bei dem man Polyisocyanate der Diphenylmethanreihe nach dem Verfahren nach Anspruch 4 herstellt und anschließend mit Polyetherpolyolen und / oder Polyesterpolyolen zu Polyurethanen umsetzt.

## Claims

1. Process for preparing polyamines of the diphenylmethane series, wherein
a) aniline and formaldehyde are firstly mixed in a molar ratio of from 1.5:1 to 6:1 in the absence of an acid catalyst and are reacted at temperatures of from 20°C to 100°C, forming aminal;
subsequent to the aminal formation, the water present in the aminal is at least partly removed, resulting in a reaction product having a water content of from 0 to 5% by weight, based on the aminal;
the aminal is then mixed with an acid catalyst selected from the group consisting of hydrochloric acid, sulphuric acid, phosphoric acid and methanesulphonic acid and subjected to a preliminary reaction at from 30°C to 95°C, where the water content of the resulting acidic reaction mixture is in the range from 0 to 20% by weight, based on the acidic reaction mixture, and the amount of acid catalyst is selected so that a degree of protonation of < 15% is obtained, where the degree of protonation of an n-protic acid is given by the molar ratio of the n-fold amount of acid catalyst used and the molar amount of amine functions present in the reaction mixture;
b) the temperature of the reaction mixture is brought to a temperature of from 110°C to 250°C when the ratio of the proportions by weight of p-aminobenzylaniline and 4,4'-MDA in the acidic reaction mixture goes below a value of 1.00.

2. Process according to Claim 1, wherein a degree of protonation of from 5 to 14% is set in step a).

3. Process according to either Claim 1 or 2, wherein the temperature is increased to values of from 110°C to 180°C in step b).

4. Process for preparing polyisocyanates of the diphenylmethane series, wherein polyamines of the diphenylmethane series are prepared by the process according to any of Claims 1 to 3 and the polyamines are subsequently phosgenated.

5. Process for preparing polyurethanes, wherein polyisocyanates of the diphenylmethane series are prepared by the process according to Claim 4 and subsequently reacted with polyether polyols and/or polyester polyols to give polyurethanes.

## Revendications

1. Procédé pour la préparation de polyamines de la série diphénylméthane, dans lequel
a) d'abord on mélange de l'aniline et du formaldéhyde en un rapport molaire de 1,5:1 à 6:1 en absence d'un catalyseur acide et on les met en réaction à des températures de 20 °C à 100 °C, de sorte qu'il se forme un aminal ;
à la suite de la formation de l'aminal on élimine au moins en partie l'eau contenue dans l'aminal, de sorte qu'il en résulte un produit de réaction ayant une teneur en eau de 0 à 5 % en poids, par rapport à l'aminal ;
on mélange ensuite l'aminal avec un catalyseur acide choisi dans le groupe constitué par l'acide chlorhydrique, l'acide sulfurique, l'acide phosphorique et l'acide méthanesulfonique et on le soumet à une pré-réaction à une température de 30 °C à 95 °C, la teneur en eau du mélange réactionnel acide résultant étant comprise entre 0 et 20 % en poids, par rapport au mélange réactionnel acide, et la quantité de catalyseur acide étant choisie de manière à ajuster un degré de protonation de < 15 %, le degré de protonation d'un acide à n protons résultant du rapport molaire de la quantité multiple n^{ème} de catalyseur acide utilisé et de la quantité molaire de fonctions amine présente dans le mélange réactionnel ;
b) on porte la température du mélange réactionnel à une température de 110 °C à 250 °C, lorsque le rapport des proportions en poids de p-aminobenzylaniline et 4,4'-MDA dans le mélange réactionnel s'abaisse au-dessous d'une valeur de 1,00.

2. Procédé selon la revendication 1, dans lequel on ajuste dans l'étape a) un degré de protonation de 5 à 14 %.

3. Procédé selon l'une quelconque des revendications 1 et 2, dans lequel on élève la température dans l'étape b) jusqu'à des valeurs de 110 °C à 180 °C.

4. Procédé pour la préparation de polyisocyanates de la série diphénylméthane, dans lequel on prépare conformément au procédé selon l'une quelconque des revendications 1 à 3 des polyamines de la série diphénylméthane et ensuite on soumet les polyamines à une phosgénation.

5. Procédé pour la préparation de polyuréthanes, dans lequel on prépare conformément au procédé selon la revendication 4 des polyisocyanates de la série diphénylméthane et ensuite on les fait réagir avec des polyétherpolyols et/ou des polyesterpolyols pour obtenir des polyuréthanes.
